# EUROPÄISCHE PATENTSCHRIFT

(11) **EP 3 795 191 B1**
(45) Veröffentlichungstag und Bekanntmachung des Hinweises auf die Patenterteilung: **20.12.2023**
(21) Anmeldenummer: 20196647.0
(22) Anmeldetag: 17.09.2020
(51) Int. Cl.: A61M 1/34

(54) **BLUTBEHANDLUNGSVORRICHTUNG MIT AUTOMATISCHER VERRINGERUNG EINER SUBSTITUTIONSLÖSUNGSFLUSSRATE**
BLOOD TREATMENT DEVICE WITH AUTOMATIC REDUCTION OF A SUBSTITUTIONAL SOLUTION FLOW RATE
DISPOSITIF DE TRAITEMENT DU SANG À RÉDUCTION AUTOMATIQUE D'UN DÉBIT DE SOLUTION DE SUBSTITUTION

(30) Priorität: 17.09.2019 DE 102019124990
(43) Veröffentlichungstag der Anmeldung: 24.03.2021
(73) Patentinhaber: B. Braun Avitum AG, 34212 Melsungen (DE)
(72) Erfinder: GOLARITS, István, 1073 Budapest (HU); Osztódi, Tibor, 1031 Budapest (HU); TÉNYI, Botond, 1037 Budapest (HU)
(74) Vertreter: Winter, Brandl - Partnerschaft mbB

(56) Entgegenhaltungen:
- EP-B1- 0 829 265
- US-A1- 2016 213 829

## Beschreibung

Die vorliegende Offenbarung betrifft eine Blutbehandlungsvorrichtung, insbesondere Dialysemaschine, zur Verwendung in (kontinuierlichen) Blutbehandlungs-/ Dialysetherapien, insbesondere Nierenersatztherapien, mit: einem extrakorporalen Blutkreislauf, einem Dialysator und einem Dialysierflüssigkeitskreislauf, wobei der extrakorporale Blutkreislauf und der Dialysierflüssigkeitskreislauf über eine in dem Dialysator vorgesehene (semipermeable) Membran, über welche Blut (unter Verwendung einer Dialysierflüssigkeitslösung) gefiltert werden kann, voneinander getrennt sind; einer Vielzahl von Pumpen, darunter zumindest: eine Substitutionslösungspumpe, welche eingerichtet ist, eine Substitutionslösung dem extrakorporalen Blutkreislauf vor und/oder nach dem Dialysator zuzuführen, und vorzugsweise eine Ultrafiltratpumpe, welche in dem Dialysierflüssigkeitskreislauf nach dem Dialysator/ stromabwärts des Dialysators vorgesehen ist; und einem Ultrafiltratdrucksensor, welcher eingerichtet ist, einen Druck in dem Dialysierflüssigkeitskreislauf nach dem Dialysator/ stromabwärts des Dialysators zu messen.

### Stand der Technik

Aus dem Stand der Technik sind bereits Blutbehandlungsvorrichtungen bekannt. Zum Beispiel offenbart die EP 0 829 265 B1 eine Blutbehandlungsvorrichtung, welche eine Schnittstelle für ein Einwegschlauchset, eine Vielzahl von Pumpen wie eine Blutpumpe, eine Spritzenpumpe, eine Ultrafiltratpumpe und eine Substitutionspumpe, Wägezellen zum Messen des Gewichts von Beuteln, welche für die Blutbehandlung erforderliche Fluide enthalten, eine Benutzeroberfläche umfassend ein Display mit Touchscreen und eine Steuereinheit zum Steuern der Prozesse der Blutbehandlungsvorrichtung aufweist. Wenn die Steuereinheit in der EP 0 829 265 B1 erfasst, dass in einem Ultrafiltratdrucksensor ein geringer Druck gemessen wird, wird ein Verstopfen des Dialysators/ des Filters angenommen und die Steuereinheit versetzt die Maschine in einen sicheren Zustand, in welchem alle vorgesehenen Pumpen gestoppt sind, und löst einen Alarm aus.

Auch offenbart ein anderes Dokument, die EP 2 504 044 B1, eine Blutbehandlungsvorrichtung mit einer Steuereinheit, welche eine Flussrate einer Substitutionslösungspumpe basierend auf einer rheologischen Last auf den Dialysator steuert/ regelt. Bei der Bestimmung der rheologischen Last werden ein zuvor berechneter Transmembrandruck und ein zuvor berechneter Flusswiderstand des Dialysators/ Filters berücksichtigt.

Weiterer Stand der Technik findet sich in der US 6 406 631 B1, der US 6,730,233 B2, der EP 1 175 917 B2, der US 2008/0251433 A1, der WO 2018/017623 A1 und der US 7,632,411 B2.

Der Stand der Technik hat dabei den Nachteil, dass der Filter entweder direkt ausgetauscht wird, wenn der Alarm von der Steuereinheit ausgegeben wird, oder dass für die Steuerung der Blutbehandlungsvorrichtung aufwändige Berechnungen, beispielsweise des Transmembrandrucks und des Flusswiderstands des Dialysators/ Filters durchgeführt werden müssen, um eine rheologische Last auf den Dialysator zu bestimmen.

Aus der US 2016/213829 A1 ist eine Blutbehandlungsvorrichtung gemäß dem Oberbegriff des Anspruchs 1 bekannt.

### Kurzbeschreibung der Offenbarung

Es ist also Aufgabe der vorliegenden Offenbarung, die Nachteile aus dem Stand der Technik zu vermeiden oder wenigstens zu mildern. Insbesondere soll die Blutbehandlungsvorrichtung so eingerichtet sein, dass sie in dem Fall eines Verstopfens des Filters oder einer Blutgerinnung die Lebensdauer des Filters/ Dialysators mit einer einfachen Steuerung verlängern kann.

Diese Aufgabe wird durch eine Blutbehandlungsvorrichtung gemäß Anspruch 1 gelöst.

Die Blutbehandlungsvorrichtung weist eine Steuereinheit auf, welche eingerichtet ist, eine Flussrate/ Förderleistung/ Fördermenge der zumindest einen Substitutionslösungspumpe automatisch zu verringern, wenn ein durch den Ultrafiltratdrucksensor gemessener Ultrafiltratdruck während einer laufenden Blutbehandlungstherapie abfällt.

In anderen Worten ist die Steuereinheit eingerichtet, (lediglich) den von dem Ultrafiltratdrucksensor gemessenen Ultrafiltratdruck während der laufenden Blutbehandlungstherapie zu überwachen. Wenn der Ultrafiltratdruck abfällt, gibt die Steuereinheit nicht unmittelbar einen Alarm aus. Die Blutbehandlungstherapie wird nicht gestoppt. Die Steuereinheit versucht an dieser Stelle, die Lebensdauer des Dialysators/ Filters zu verlängern, indem sie die Flussrate/ Förderleistung/ Fördermenge der zumindest einen Substitutionslösungspumpe verringert.

Die Steuereinheit ist eingerichtet, die Flussrate der zumindest einen Substitutionslösungspumpe derart zu verringern/ zu ändern/ einzustellen/ zu steuern, dass ein stabiler Ultrafiltratdruck erreicht wird, welcher zumindest einen vorbestimmten Abstand zu einem (vorbestimmten) Niederdruck-Alarm-Grenzwert einhält. Der vorbestimmte Niederdruck-Alarm-Grenzwert stellt einen Grenzwert dar. Wenn der Ultrafiltratdruck unter diesen Grenzwert fällt, also ein Niederdruck in dem Ultrafiltratdruck vorliegt, wird ein Alarm ausgegeben.

Bevorzugt ist die Steuereinheit eingerichtet, festzustellen, dass ein Verstopfen des Dialysator/ des Filters/ der Membran oder eine Blutgerinnung vorliegt, und zwar einzig/ allein/ ausschließlich vor dem Hintergrund/ auf der Grundlage, dass der durch den Ultrafiltratdrucksensor gemessene Ultrafiltratdruck während der laufenden Blutbehandlungstherapie abfällt.

In anderen Worten werden bei der Feststellung, dass ein Verstopfen des Dialysators oder eine Blutgerinnung vorliegt, ein Transmembrandruck und/oder ein Flusswiderstand des Dialysators bevorzugt nicht mit einbezogen.

In anderen Worten ist die Steuereinheit bevorzugt eingerichtet, die Einstellung/ Verringerung der Flussrate der Substitutionslösungspumpe einzig/ allein/ ausschließlich auf der Grundlage des Ultrafiltratdrucks vorzunehmen und nicht auf der Grundlage des Transmembrandrucks oder des Flusswiderstands des Dialysators oder einer anderen Einflussgröße.

Bevorzugt ist die Steuereinheit eingerichtet, in dem Fall, in welchem ein Verstopfen des Dialysators oder eine Blutgerinnung vorliegt, den Ultrafiltratdruck einzig/ allein/ ausschließlich durch ein Ändern der Flussrate der zumindest einen Substitutionslösungspumpe zu steuern/ einzustellen.

Es hat sich als vorteilhaft herausgestellt, wenn der stabile Ultrafiltratdruck mindestens 50 mmHg über dem Niederdruck-Alarm-Grenzwert ist.

Es ist zweckmäßig, wenn die Blutbehandlungsvorrichtung eine Benutzeroberfläche umfassend ein Display mit Touch-Screen aufweist und die Steuereinheit eingerichtet ist, eine Warnung auf dem Display anzuzeigen, wenn der Ultrafiltratdruck während der Therapie abfällt und die Flussrate der Substitutionslösungspumpe geändert/ verringert wird.

Bevorzugt ist die Steuereinheit eingerichtet, die Blutbehandlungstherapie zu stoppen und einen Alarm auszugeben, wenn eine Flussrate von zumindest einer Substitutionslösungspumpe unter einen vorbestimmten Wert fällt.

Der vorbestimmte Wert ist dabei bevorzugt zwischen 25 ml/h und 75 ml/h, insbesondere bei etwa/ näherungsweise 50 ml/h eingestellt.

Ferner ist es von Vorteil, wenn die Steuereinheit eingerichtet ist, wenn der Ultrafiltratdruck in einen normalen Druckbereich (Ausgangsdruck) zurückkehrt, die Flussrate der Substitutionslösungspumpe zu erhöhen.

In vorteilhafter Weise ist die Steuereinheit eingerichtet, bei einem mehrmaligen bzw. wiederholten Abfallen des Ultrafiltratdrucks die Flussrate der zumindest einen Substitutionslösungspumpe mehrmalig bzw. wiederholt zu verringern, um so den Ultrafiltratdruck bei einem vorbestimmten stabilen Ultrafiltratdruck zu halten oder wieder auf den vorbestimmten stabilen Ultrafiltratdruck einzustellen, und zwar solange bis die Flussrate der zumindest einen Substitutionslösungspumpe unter einen vorbestimmten Wert fällt.

Es ist zweckmäßig, wenn mehrere, insbesondere zwei, Substitutionslösungspumpen in der Blutbehandlungsvorrichtung vorgesehen sind und die Steuereinheit eingerichtet ist, die Blutbehandlungstherapie zu stoppen und einen Alarm auszugeben, wenn eine Flussrate von zumindest einer aus den Substitutionslösungspumpen unter einen vorbestimmten Wert fällt.

Bevorzugt weist die Blutbehandlungsvorrichtung eine Wägevorrichtung, insbesondere Wägezelle, zum Messen des Gewichts eines Beutels, insbesondere Einwegbeutels, welcher eine für die Blutbehandlung erforderliche Lösung, beispielsweise eine Substitutionslösung, enthält, auf.

Es ist zweckmäßig, wenn der extrakorporale Blutkreislauf und der Dialysierflüssigkeitskreislauf als Einwegschläuche ausgebildet sind, welche an einer an der Dialysemaschine vorgesehenen Schnittstelle aufgenommen sind.

Bevorzugt umfasst die Vielzahl von Pumpen neben der Substitutionslösungspumpe und der Ultrafiltratpumpe zumindest eine Blutpumpe und eine Spritzenpumpe.

Die Blutbehandlungsvorrichtung weist ferner vorzugsweise ein Bar-Code-Lesegerät auf, welches eingerichtet ist, auf Einwegartikel wie etwa Einwegschläuche aufgebrachte Bar-Codes zu lesen.

Die Blutbehandlungsvorrichtung ist bevorzugt zur drahtgebundenen Kommunikation eingerichtet.

Die Steuereinheit der Blutbehandlungsvorrichtung ist vorzugsweise als zumindest ein Prozessor, vorzugsweise mehrere Prozessoren, ausgebildet.

Mit anderen Worten betrifft die Offenbarung eine Dialysemaschine. Die Dialysemaschine enthält ein Bar-Code-Lesegerät. Weiterhin enthält die Dialysemaschine eine Benutzeroberfläche bzw. ein Display mit Touch-Screen. Die Dialysemaschine weist ferner eine Schnittstelle/ Interface für ein Einwegschlauchset auf, welches eine Blutseite und eine Dialysierflüssigkeitsseite enthält, die voneinander durch eine (semi-) permeable/ durchlässige Membran getrennt sind, um Blut unter Verwendung einer Dialysierflüssigkeitslösung/ Dialyselösung zu filtern. Eine Substitutionslösung/ Ersatzlösung wird der Blutseite vor/nach einem Dialysator zugeführt. Die Dialysemaschine weist eine Blutpumpe, eine Spritzenpumpe, eine Ultrafiltratpumpe, eine Substitutionslösungspumpe etc. auf. Die Dialysemaschine ist zur drahtgebundenen Kommunikation eingerichtet/ weist verdrahtete bzw. drahtgebundene Kommunikationseinrichtungen auf. Die Dialysemaschine zeichnet sich durch eine Software aus, welche besonders für eine Verwendung in kontinuierlichen Dialysetherapien, beispielsweise Nierenersatztherapien geeignet ist. Die Software läuft dabei auf einer Vielzahl von Prozessoren innerhalb der Dialysemaschine. Weiterhin weist die Dialysemaschine eine Energieverwaltungseinrichtung (integrierter Schaltkreis) auf. Die Dialysemaschine enthält darüber hinaus Wägevorrichtungen, insbesondere Wägezellen, welche das Gewicht von Einwegbeuteln messen, welche die für die Dialysetherapie erforderlichen Fluide (z.B. Dialysierflüssigkeitslösung, Substitutionslösung) enthalten.

Die Blutbehandlungsvorrichtung/ die Steuereinheit derselben stellt eine automatische Verringerung einer Substitutionslösungsflussrate bereit. Die Steuereinheit ist eingerichtet, das Substitutionsfluidvolumen anzupassen, wenn eine Verstopfung des Dialysators/ Filters bzw. eine Blutgerinnung auftritt. Wenn der Ultrafiltratdruck während einer Therapie abfällt, verringert die Steuereinheit/ das System automatisch die Flussrate des Substitutionsfluids/ der Substitutionslösung, um einen stabilen Ultrafiltratdruck zu erreichen, welcher zumindest 50 mmHg über einem Ultrafiltrat-Niederdruck-Alarm-Grenzwert ist. In diesem Fall wird eine Warnung auf dem Display/ Touch-Screen angezeigt. Die Steuerung der Zugabe der Substitutionslösung kann auch eine Verteilung der Zufuhr der Substitutionslösung stromauf und stromab des Dialysators umfassen (Prädilution und Postdilution). Falls anwendbar, wird demnach die Postdilution-Flussrate erhöht und falls erforderlich wird auch der Prädilution-Fluss erhöht. Wenn die Flussrate von einer der (Substitutionslösungs-) Pumpen unter 50 ml/h fällt, stoppt die Maschine die Behandlung und gibt einen Alarm mit geringer Priorität aus. Wenn der Ultrafiltratdruck in einen normalen Druckbereich zurückkehrt, kompensiert das System/ die Steuereinheit das fehlende Fluidvolumen, indem die Substitutionslösungsflussrate erhöht wird. Diese Steuerung/ diese Eigenschaft kann von einem Servicetechniker aktiviert und deaktiviert werden.

### Kurzbeschreibung der Figuren

Die Offenbarung wird nachfolgend mit Hilfe von Figuren weiter erläutert. Es zeigen:
- Fig. 1: eine schematische Ansicht einer Blutbehandlungsvorrichtung gemäß der vorliegenden Offenbarung;
- Fig. 2: ein Flussdiagramm, welches die offenbarungsgemäße, in der Steuereinheit ablaufende automatische Verringerung einer Substitutionslösungsflussrate veranschaulicht; und
- Fig. 3: ein Diagramm, in welchem ein zeitlicher Verlauf der Substitutionslösungsflussrate und eines Ultrafiltratdrucks gemäß der vorliegenden Offenbarung dargestellt ist.

### Figurenbeschreibung

Die Figuren sind lediglich schematischer Natur und dienen ausschließlich dem Verständnis der vorliegenden Offenbarung. Gleiche Elemente sind dabei mit denselben Bezugszeichen versehen.

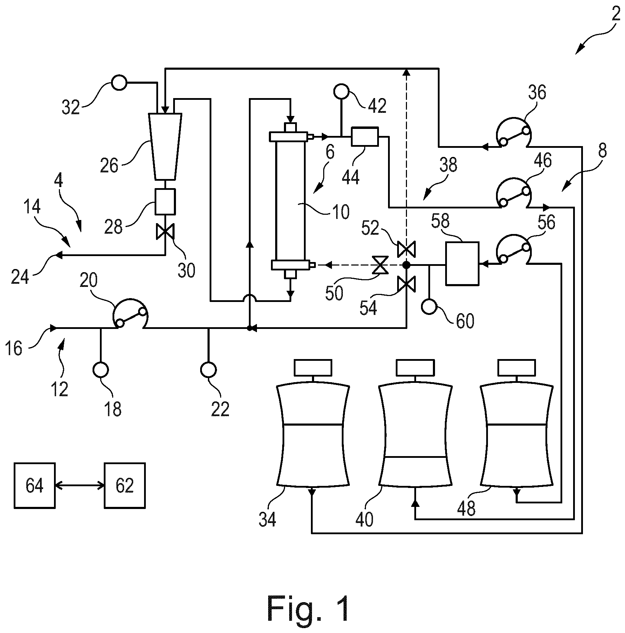

Fig. 1 zeigt eine schematische Ansicht einer extrakorporalen Blutbehandlungsvorrichtung (Dialysemaschine) 2. Die Blutbehandlungsvorrichtung 2 ist grundsätzlich eingerichtet, sowohl in kontinuierlichen als auch in intermittierenden Blutbehandlungstherapien, insbesondere Nierenersatztherapien, verwendet zu werden. Die Blutbehandlungsvorrichtung 2 ist insbesondere als eine Akutdialysemaschine bzw. als ein Akut-Dialysegerät ausgebildet und ist somit im Wesentlichen zur Verwendung auf Intensivstationen bei überwiegend instabilen Patienten vorbereitet. Mit der Blutbehandlungsvorrichtung 2 der vorliegenden Offenbarung können grundsätzlich eine Vielzahl von verschiedenen Blutbehandlungstherapien (z.B. langsame kontinuierliche Ultrafiltration (SCUF), kontinuierliche venös-venöse Hämofiltration (CWH), kontinuierliche venös-venöse Hämodialyse (CWHD), kontinuierliche venös-venöse Hämodiafiltration (CWHDF), therapeutischer Plasmaaustauch (TPE), etc.), Dilutionsmodi (z.B. Prädilution, Postdilution, Prä- und Postdilution), sowie Antikoagulationsarten (z.B. keine, Heparin, Citrat, etc.) durchgeführt werden.

Die Blutbehandlungsvorrichtung 2 weist grundsätzlich einen extrakorporalen Kreislauf 4, einen Dialysator (Hämofilter) 6 und einen Dialysierflüssigkeitskreislauf 8 auf. Der extrakorporale Kreislauf 4 und der Dialysierflüssigkeitskreislauf 8 sind über eine in dem Dialysator 6 vorgesehene Membran 10, über welche Blut unter Verwendung einer Dialysierflüssigkeitslösung oder ohne Verwendung einer Dialysierflüssigkeitslösung gefiltert werden kann, voneinander getrennt.

Der extrakorporale Kreislauf 4 umfasst einen arteriellen Abschnitt 12 und einen venösen Abschnitt 14. Dabei ist grundsätzlich vorgesehen, dass der arterielle Abschnitt 12, insbesondere ein Ende desselben, an eine Arterie eines Patienten, insbesondere eines Intensivpatienten, angeschlossen bzw. mit derselben verbunden werden soll. Weiterhin ist vorgesehen, dass der venöse Abschnitt 14, insbesondere ein Ende desselben, an eine Vene eines Patienten, insbesondere eines Intensivpatienten, angeschlossen bzw. mit derselben verbunden werden soll.

Der arterielle Abschnitt 12 weist ausgehend von einem arteriellen Ende 16 in einer Blut-Strömungsrichtung hin zu dem Dialysator 6 einen arteriellen Drucksensor 18, eine (arterielle) Blutpumpe 20 und einen Dialysatoreingangsdrucksensor 22 auf. Der venöse Abschnitt 14 weist ausgehend von dem Dialysator 6 in einer Blut-Strömungsrichtung hin zu einem venösen Ende 24 eine venöse Expansionskammer bzw. Luftfalle 26, einen Sicherheitsluftdetektor 28 und ein Sicherheitsventil 30 auf. An/ hinter der venösen Expansionskammer 26 kann ein venöser Druck über einen venösen Drucksensor 32 gemessen werden.

Wie aus Fig. 1 hervorgeht, ist die venöse Expansionskammer 26 mit einem Substitutionslösungsbeutel/ -behälter 34 verbunden. Eine Substitutionslösungspumpe 36 ist dabei vorgesehen und eingerichtet, eine Substitutionslösung aus dem Substitutionslösungsbeutel 34 in den extrakorporalen Blutkreislauf 4, insbesondere in den venösen Abschnitt 14 desselben (in die venöse Expansionskammer 26) zu pumpen.

Der Dialysierflüssigkeitskreislauf 8 weist zumindest einen Abfluss 38 für Ultrafiltrat/ verbrauchte Dialysierflüssigkeit (Dialysat)/ eine sonstige Flüssigkeit auf. Grundsätzlich kann das Ultrafiltrat/ Dialysat/ die sonstige Flüssigkeit über den Abfluss 38 von dem Dialysator 6 hin zu einem Sammelbeutel/ -behälter 40 für Ultrafiltrat/ Dialysat/ etc. fließen. In dem Abfluss 38 sind ausgehend von dem Dialysator 6 in einer Strömungsrichtung hin zu dem Sammelbeutel 40 ein Ultrafiltratdrucksensor 42, ein Blutleckdetektor 44 und eine Ultrafiltratpumpe 46 angeordnet bzw. vorgesehen.

Wie aus Fig. 1 weiter hervorgeht, ist neben dem Substitutionslösungsbeutel 34 und dem Sammelbeutel 40 noch ein weiterer Beutel/ Behälter 48 vorgesehen. Der Beutel 48 kann in Abhängigkeit von der gewünschten durchzuführenden Blutbehandlungstherapie beispielsweise eine Substitutionslösung /-flüssigkeit oder eine Dialysierflüssigkeit enthalten.

Wenn beispielsweise mit der extrakorporalen Blutbehandlungsvorrichtung 2 eine Hämodialyse-/ eine Hämodiafiltrationsbehandlung etc. durchgeführt werden soll, also eine Blutbehandlungstherapie, in welcher Dialysierflüssigkeit durch den Dialysator 6 strömt und somit ein Stofftransport von dem extrakorporalen Kreislauf 4 zu dem Dialysierflüssigkeitskreislauf 8 sowohl über Diffusion als auch über Konvektion erfolgt, enthält der Beutel 48 Dialysierflüssigkeit. Wird nun ein erstes Ventil 50 geöffnet und werden sowohl ein zweites Ventil 52 als auch ein drittes Ventil 54 geschlossen, kann die Dialysierflüssigkeit über eine Pumpe 56 zum Dialysator 6 gepumpt werden.

Soll alternativ mit der extrakorporalen Blutbehandlungsvorrichtung 2 beispielsweise eine Hämofiltration etc. durchgeführt werden, also eine Blutbehandlungstherapie, in welcher keine Dialysierflüssigkeit durch den Dialysator 6 strömt und somit ein Stofftransport von dem extrakorporalen Kreislauf 4 zu dem Dialysierflüssigkeitskreislauf 8 lediglich über Konvektion/ Filtration erfolgt, kann der Beutel 48 eine Substitutionslösung enthalten.

Werden nun das erste Ventil 50 und das zweite Ventil 52 geschlossen und wird das dritte Ventil 54 geöffnet, kann die Substitutionslösung aus dem Beuel 48 in den arteriellen Abschnitt 12 des extrakorporalen Kreislaufs 4 gepumpt werden (Prädilution). Werden das erste Ventil 50 und das dritte Ventil 54 geschlossen und wird das zweite Ventil 52 geöffnet, kann die Substitutionslösung aus dem Beuel 48 in den venösen Abschnitt 14 des extrakorporalen Kreislaufs 4 gepumpt werden (Postdilution). Wird das erste Ventil 50 geschlossen und werden das zweite Ventil 52 und das dritte Ventil 54 geöffnet, kann die Substitutionslösung aus dem Beutel 48 sowohl in den arteriellen Abschnitt 12 als auch in den venösen Abschnitt 14 des extrakorporalen Kreislaufs gepumpt werden (Prä- und Postdilution). Eine Prä- und Postdilution kann gemäß der vorliegenden Offenbarung auch dadurch erreicht werden, dass die Substitutionslösung aus dem Substitutionslösungsbeutel 34 mittels der Substitutionslösungspumpe 36 in den venösen Abschnitt 14 des extrakorporalen Kreislaufs 4 gepumpt wird (Postdilution), und dass gleichzeitig die Substitutionslösung aus dem Beutel 48 mittels der Pumpe (Substitutionslösungspumpe) 56 in den arteriellen Abschnitt 12 des extrakorporalen Kreislaufs 4 gepumpt wird (Prädilution).

Wie aus Fig. 1 weiter hervorgeht, sind zwischen der Pumpe 56 und der Ventilanordnung bestehend aus dem ersten Ventil 50, dem zweiten Ventil 52 und dem dritten Ventil 54 ein Flüssigkeitswärmer 58 und ein Drucksensor 60 vorgesehen.

Die extrakorporale Blutbehandlungsvorrichtung 2 weist weiterhin eine Steuereinheit 62 auf, welche Informationen von den in der Blutbehandlungsvorrichtung 2 vorgesehenen Sensoren erhält, und welche die in der Blutbehandlungsvorrichtung 2 vorgesehenen Aktoren ansteuert. Offenbarungsgemäß wird somit insbesondere eine softwareunterstützte Therapie bereitgestellt. Die Steuereinheit 62 erhält dabei insbesondere Informationen von dem arteriellen Drucksensor 18, dem Dialysatoreingangsdrucksensor 22, dem Sicherheitsluftdetektor 28, dem venösen Drucksensor 32, dem Ultrafiltratdrucksensor 42, dem Blutleckdetektor 44, dem Drucksensor 60, etc. Die Steuereinheit 62 steuert insbesondere die Blutpumpe 20, das Sicherheitsventil 30, die Substitutionslösungspumpe 36, die Ultrafiltratpumpe 46, das erste Ventil 50, das zweite Ventil 52, das dritte Ventil 54, die Pumpe 56, den Flüssigkeitswärmer 58, etc. an. Weiterhin ist die Steuereinheit 62 im Austausch mit einer als Display mit Touch-Screen ausgebildeten Benutzeroberfläche 64. Beispielsweise kann die Steuereinheit 62 eingerichtet sein, eine Warnung auf der Benutzeroberfläche 64 anzuzeigen. Weiterhin können von einem Nutzer/ Anwender auf der Benutzeroberfläche 64 eingegebene Informationen an die Steuereinheit 62 weitergegeben werden.

Die vorliegende Offenbarung betrifft im Wesentlichen eine durch die Steuereinheit 62 vorgenommenen Steuerung. Wenn die Steuereinheit 62 eine Information von dem Ultrafiltratdrucksensor 42 erhält, dass der Ultrafiltratdruck während einer laufenden Blutbehandlungstherapie abfällt, steuert die Steuereinheit 62 gemäß der vorliegenden Offenbarung die Substitutionslösungspumpe 36 und/oder die Pumpe 56 an (wenn in dem Beutel 48 Substitutionslösung enthalten ist und die Pumpe 56 eine (zweite) Substitutionslösungspumpe ist), eine Flussrate/ einen Fluss/ einen Volumenstrom durch die entsprechende Pumpe 36 und/oder 56 automatisch zu verringern.

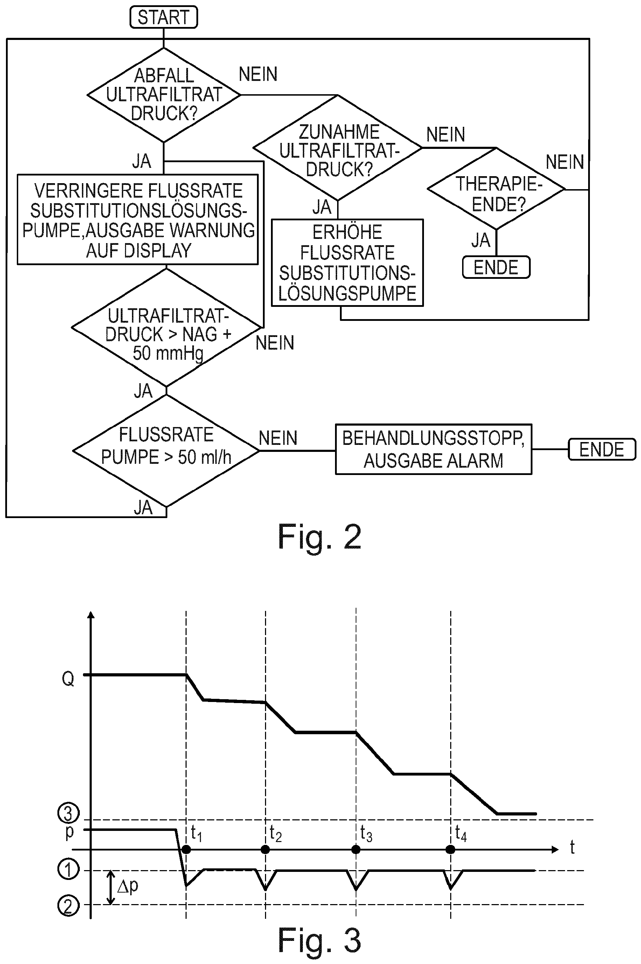

Fig. 2 zeigt den offenbarungsgemäßen Ablauf einer automatischen Verringerung einer Substitutionslösungsflussrate, welcher wie folgt zusammengefasst werden kann: Die Steuereinheit 62 der vorliegenden Offenbarung erhält grundsätzlich Informationen von dem Ultrafiltratdrucksensor 42, welcher einen Druck in dem Dialysierflüssigkeitskreislauf 8 stromabwärts des Dialysators 6 / nach dem Dialysator 6 überwacht. Wenn die Steuereinheit 62 feststellt, dass der Ultrafiltratdruck während einer laufenden Blutbehandlungstherapie abfällt, schlussfolgert sie, dass ein Verstopfen des Dialysators 6 oder eine Blutgerinnung vorliegt. Um in einem solchen Fall die Lebensdauer des Dialysators/ Filters 6 zu verlängern, verringert die Steuereinheit 62 eine Flussrate zumindest einer Substitutionslösungspumpe 36, 56, welche grundsätzlich eine Substitutionslösung in den extrakorporalen Blutkreislauf 4 vor und/oder nach dem Dialysator 6 pumpt. Anschließend prüft die Steuereinheit 62, ob ein stabiler Ultrafiltratdruck durch diese Maßnahme erreicht werden konnte. Der stabile Ultrafiltratdruck sollte dabei zumindest 50 mmHg über einem vorbestimmten Niederdruck-Alarm-Grenzwert (Grenzwert, unter welchem ein Alarm ausgelöst wird) sein. Anschließend prüft die Steuereinheit 62, ob die Flussrate/ Förderleistung/ Fördermenge von der Pumpe 56 und/oder der Substitutionslösungspumpe 36 größer als 50 ml/h ist. Falls die Flussrate von einer der Substitutionslösungspumpen nicht größer als 50 ml/h ist, erfolgt ein Behandlungsstopp, ein Alarm wird ausgegeben und die vorliegende Routine endet. Falls die Flussrate von allen Substitutionslösungspumpen 36, 56 über 50 ml/h ist, überwacht die Steuereinheit 62, ob in dem Ultrafiltratdruck ein weiterer Abfall oder eine Zunahme auftritt. Tritt ein weiterer Abfall auf, wird die Flussrate der Substitutionslösungspumpe(n) 36, 56 weiter verringert. Tritt eine Zunahme auf, wird die Flussrate der Substitutionslösungspumpe(n) 36, 56 entsprechend erhöht. Die vorliegende Routine endet auch, wenn ein Therapieende vorliegt.

Die offenbarungsgemäße Steuerung wird im Detail noch unter Bezugnahme auf das in Fig. 3 dargestellte Diagramm erläutert. Hier wird deutlich, dass zunächst die Substitutionslösungsflussrate Q konstant verläuft, und dass auch der Ultrafiltratdruck p konstant verläuft. Die Substitutionslösungsflussrate Q kann grundsätzlich die Flussrate der Substitutionspumpe 36 sein. Weiterhin kann die Substitutionslösungsflussrate Q grundsätzlich auch die Flussrate der Pumpe 56 sein, insbesondere wenn die Pumpe 56 als Substitutionslösungspumpe arbeitet.

Bei Zeitpunkt t1 wurde von der Steuereinheit 62 (auf der Grundlage der von dem Ultrafiltratdrucksensor 42 übermittelten Informationen) erfasst, dass der Ultrafiltratdruck während der laufenden Blutbehandlungstherapie abgefallen ist, und die Steuereinheit 62 steuert die Substitutionslösungspumpe 36 oder die Pumpe 56 oder beide Pumpen 36, 56 an, die Substitutionslösungsflussrate Q zu verringern. Gleichzeitig gibt die Steuereinheit 62 eine Warnung auf der Benutzeroberfläche 64 aus. Wie aus Fig. 3 hervorgeht, wird durch die Verringerung der Substitutionslösungsflussrate Q ab Zeitpunkt t1 erreicht, dass sich der Ultrafiltratdruck auf einen stabilen Wert/ einen stabilen Ultrafiltratdruck einstellt (in Fig. 3 mit ① gekennzeichnet). Dieser stabile Ultrafiltratdruck hat vorliegend eine Abstand Δp zu einem Niederdruck-Alarm-Grenzwert, welcher in Fig. 3 mit ② gekennzeichnet ist. Gemäß der vorliegenden Offenbarung beträgt Δp bevorzugt zumindest 50 mmHg, das heißt der stabile Ultrafiltratdruck ① ist mindestens um 50 mmHg größer als der Niederdruck-Alarm-Grenzwert ②.

Insbesondere hat sich gemäß der vorliegenden Offenbarung herausgestellt, dass, wenn der angesprochene Mindestabstand Δp zwischen dem stabilen Ultrafiltratdruck ① und dem Niederdruck-Alarm-Grenzwert ② eingehalten wird, bei einem erneuten Abfall des Ultrafiltratdrucks, welcher gemäß Fig. 3 bei t2 von der Steuereinheit 62 festgestellt wurde, die Steuereinheit 62 rechtzeitig reagieren kann und die Substitutionslösungsflussrate Q weiter verringern kann, und zwar bevor der Niederdruck-Alarm-Grenzwert ② unterschritten wird. Somit wird auch bei einem erneuten Abfall des Ultrafiltratdrucks gemäß der vorliegenden Offenbarung erreicht, dass sich der Ultrafiltratdruck durch eine rechtzeitige Reaktion der Steuereinheit 62 wieder auf den stabilen Ultrafiltratdruck ① einstellen kann. In anderen Worten wird durch die offenbarungsgemäße Steuerung erreicht, dass der Niederdruck-Alarm-Grenzwert ② nicht unterschritten wird. Dasselbe wie bei Zeitpunkt t2 kann bei den Zeitpunkten t3 und t4 in Fig. 3 beobachtet werden.

Die Steuereinheit 62 überprüft gemäß der offenbarungsgemäßen Steuerung weiterhin, insbesondere wenn sich der Ultrafiltratdruck wieder auf den stabilen Ultrafiltratdruck ① eingestellt hat, ob die Substitutionslösungsflussrate Q noch über einem vorbestimmten Wert ist, welcher in Fig. 3 mit ③ gekennzeichnet ist. Solange dies der Fall ist, wird offenbarungsgemäß die Blutbehandlungstherapie fortgesetzt. Erst wenn der vorbestimmte Wert ③ von zumindest einer Substitutionslösungspumpe 36, 56 unterschritten wird, erfolgt ein Behandlungsstopp und ein Alarm wird ausgegeben.

Es hat sich offenbarungsgemäß herausgestellt, dass der vorbestimmte Wert bevorzugt bei 50 ml/h einzustellen ist. Wenn die Substitutionslösungsflussrate Q von zumindest einer Substitutionslösungspumpe 36, 56 unter diesen Wert fällt, ist insbesondere davon auszugehen, dass der Dialysator 6 in der Zwischenzeit derart verstopft wurde, dass die Blutbehandlungstherapie nicht mehr fortgesetzt werden sollte. Insbesondere sollte vor einer Wiederaufnahme der Blutbehandlungstherapie der Dialysator 6 ausgewechselt werden.

### Bezugszeichenliste

- 2: Blutbehandlungsvorrichtung! Dialysemaschine
- 4: extrakorporaler Kreislauf
- 6: Dialysator
- 8: Dialysierflüssigkeitskreislauf
- 10: Membran
- 12: arterieller Abschnitt
- 14: venöser Abschnitt
- 16: arterielles Ende
- 18: arterieller Drucksensor
- 20: (arterielle) Blutpumpe
- 22: Dialysatoreingangsdrucksensor
- 24: venöses Ende
- 26: venöse Expansionskammer/ Luftfalle
- 28: Sicherheitsluftdetektor
- 30: Sicherheitsventil
- 32: venöser Drucksensor
- 34: Substitutionslösungsbeutel
- 36: Substitutionslösungspumpe
- 38: Abfluss
- 40: Sammelbeutel
- 42: Ultrafiltratdrucksensor
- 44: Blutleckdetektor
- 46: Ultrafiltratpumpe
- 48: Beutel
- 50: erstes Ventil
- 52: zweites Ventil
- 54: drittes Ventil
- 56: Pumpe
- 58: Flüssigkeitswärmer
- 60: Drucksensor
- 62: Steuereinheit
- 64: Benutzeroberfläche

## Patentansprüche

1. Blutbehandlungsvorrichtung (2), insbesondere Dialysemaschine, zur Verwendung in Blutbehandlungstherapien, insbesondere Nierenersatztherapien, mit:
einem extrakorporalen Blutkreislauf (4), einem Dialysator (6) und einem Dialysierflüssigkeitskreislauf (8), wobei der extrakorporale Blutkreislauf (4) und der Dialysierflüssigkeitskreislauf (8) über eine in dem Dialysator (6) vorgesehene Membran (10), über welche Blut gefiltert werden kann, voneinander getrennt sind;
zumindest einer Substitutionslösungspumpe (36, 56), welche eingerichtet ist, eine Substitutionslösung dem extrakorporalen Blutkreislauf (4) vor und/oder nach dem Dialysator (6) zuzuführen;
einem Ultrafiltratdrucksensor (42), welcher eingerichtet ist, einen Druck in dem Dialysierflüssigkeitskreislauf (8) nach dem Dialysator (6) zu messen; und
einer Steuereinheit (62), welche eingerichtet ist,
eine Flussrate der zumindest einen Substitutionslösungspumpe (36, 56) automatisch zu verringern, wenn ein durch den Ultrafiltratdrucksensor (42) gemessener Ultrafiltratdruck während einer laufenden Blutbehandlungstherapie abfällt,
**dadurch gekennzeichnet, dass**
die Steuereinheit (62) eingerichtet ist, die Flussrate der zumindest einen Substitutionslösungspumpe (36, 56) derart zu verringern, dass ein stabiler Ultrafiltratdruck erreicht wird, welcher zumindest einen vorbestimmten Abstand zu einem Niederdruck-Alarm-Grenzwert einhält.

2. Blutbehandlungsvorrichtung (2) nach Anspruch 1, **dadurch gekennzeichnet, dass** die Steuereinheit (62) eingerichtet ist, festzustellen, dass ein Verstopfen des Dialysators (6) oder eine Blutgerinnung vorliegt, und zwar einzig, allein bzw. ausschließlich auf der Grundlage, dass der durch den Ultrafiltratdrucksensor (62) gemessene Ultrafiltratdruck während der laufenden Blutbehandlungstherapie abfällt.

3. Blutbehandlungsvorrichtung (2) nach Anspruch 2, **dadurch gekennzeichnet, dass** bei der Feststellung, dass ein Verstopfen des Dialysators (6) oder eine Blutgerinnung vorliegt, ein Transmembrandruck und/oder ein Flusswiderstand des Dialysators (6) nicht mit einbezogen werden.

4. Blutbehandlungsvorrichtung (2) nach einem der vorhergehenden Ansprüche, **dadurch gekennzeichnet, dass** die Steuereinheit (62) eingerichtet ist, in dem Fall, in welchem ein Verstopfen des Dialysators (6) oder eine Blutgerinnung vorliegt, den Ultrafiltratdruck einzig, allein bzw. ausschließlich durch ein Ändern der Flussrate der zumindest einen Substitutionslösungspumpe (36, 56) einzustellen.

5. Blutbehandlungsvorrichtung (2) nach einem der vorhergehenden Ansprüche, **dadurch gekennzeichnet, dass** der stabile Ultrafiltratdruck mindestens 50 mmHg über dem Niederdruck-Alarm-Grenzwert ist.

6. Blutbehandlungsvorrichtung (2) nach einem der vorhergehenden Ansprüche, **dadurch gekennzeichnet, dass** die Blutbehandlungsvorrichtung (2) eine Benutzeroberfläche (64) umfassend ein Display mit Touch-Screen aufweist und die Steuereinheit (62) eingerichtet ist, eine Warnung auf dem Display anzuzeigen, wenn der Ultrafiltratdruck während der Therapie abfällt und die Flussrate der Substitutionslösungspumpe (36, 56) verringert wird.

7. Blutbehandlungsvorrichtung (2) nach einem der vorhergehenden Ansprüche, **dadurch gekennzeichnet, dass** die Steuereinheit (62) eingerichtet ist, die Blutbehandlungstherapie zu stoppen und einen Alarm auszugeben, wenn eine Flussrate von zumindest einer Substitutionslösungspumpe (36, 56) unter einen vorbestimmten Wert fällt.

8. Blutbehandlungsvorrichtung (2) nach Anspruch 7, **dadurch gekennzeichnet, dass** der vorbestimmte Wert zwischen 25 ml/h und 75 ml/h, insbesondere auf etwa 50 ml/h, eingestellt ist.

9. Blutbehandlungsvorrichtung (2) nach einem der vorhergehenden Ansprüche, **dadurch gekennzeichnet, dass** die Steuereinheit (62) eingerichtet ist, wenn der Ultrafiltratdruck in einen normalen Druckbereich zurückkehrt, die Flussrate der Substitutionslösungspumpe (36, 56) zu erhöhen.

10. Blutbehandlungsvorrichtung (2) nach einem der vorhergehenden Ansprüche, **dadurch gekennzeichnet, dass** die Steuereinheit (62) eingerichtet ist, bei einem mehrmaligen bzw. wiederholten Abfallen des Ultrafiltratdrucks die Flussrate der zumindest einen Substitutionslösungspumpe (36, 56) mehrmalig bzw. wiederholt zu verringern, um so den Ultrafiltratdruck bei einem vorbestimmten stabilen Ultrafiltratdruck zu halten oder wieder auf den vorbestimmten stabilen Ultrafiltratdruck einzustellen, und zwar solange bis die Flussrate der zumindest einen Substitutionslösungspumpe (36, 56) unter einen vorbestimmten Wert fällt.

11. Blutbehandlungsvorrichtung (2) nach einem der vorhergehenden Ansprüche, **gekennzeichnet durch** mehrere, insbesondere zwei, Substitutionslösungspumpen (36, 56), wobei die Steuereinheit (62) eingerichtet ist, die Blutbehandlungstherapie zu stoppen und einen Alarm auszugeben, wenn eine Flussrate von zumindest einer aus den Substitutionslösungspumpen (36, 56) unter einen vorbestimmten Wert fällt.

## Claims

1. A blood treatment device (2), in particular a dialysis machine, for use in blood treatment therapies, particularly renal replacement therapies, the blood treatment device comprising:
an extracorporeal blood circuit (4), a dialyzer (6) and a dialysis fluid circuit (8), the extracorporeal blood circuit (4) and the dialysis fluid circuit (8) separated from each other via a membrane (10) provided in the dialyzer (6), via which blood is filterable;
at least one substitution solution pump (36, 56), which is configured to supply a substitution solution to the extracorporeal blood circuit (4) before and/or after the dialyzer (6);
an ultrafiltrate pressure sensor (42) configured to measure a pressure in the dialysis fluid circuit (8) after the dialyzer; and
a control unit (62) configured
to automatically reduce a flow rate of the at least one substitution solution pump (36, 56) when an ultrafiltrate pressure measured by the ultrafiltrate pressure sensor (42) drops during an ongoing blood treatment therapy,
**characterized in that**
the control unit (62) is configured to reduce the flow rate of the at least one substitution solution pump (36, 56) such that a stable ultrafiltrate pressure is achieved, which keeps at least a predetermined distance from a low-pressure warning threshold.

2. The blood treatment device (2) according to claim 1, **characterized in that** the control unit (62) is configured to determine that clogging of the dialyzer (6) or blood clotting is present solely and/or exclusively on the basis of the ultrafiltrate pressure measured by the ultrafiltrate pressure sensor (62) dropping during an ongoing blood treatment therapy.

3. The blood treatment device (2) according to claim 2, **characterized in that** at least one of a transmembrane pressure and a flow resistance of the dialyzer (6) is not included in determining that clogging of the dialyzer (6) or blood clotting is present.

4. The blood treatment device (2) according to any of the preceding claims, **characterized in that** the control unit (62) is configured, in case of clogging of the dialyzer (6) or blood clotting, to adjust the ultrafiltrate pressure solely and/or exclusively by changing the flow rate of the at least one substitution solution pump (36, 56).

5. The blood treatment device (2) according to any of the preceding claims, **characterized in that** the stable ultrafiltrate pressure is at least 50mmHg above the low-pressure warning threshold.

6. The blood treatment device (2) according to any of the preceding claims, **characterized in that** the blood treatment device (2) has a user interface (64) comprising a display with touch screen and the control unit (62) is arranged to display a warning on the display when the ultrafiltrate pressure drops during the therapy and the flow rate of the substitution solution pump (36, 56) is reduced.

7. The blood treatment device (2) according to any of the preceding claims, **characterized in that** the control unit (62) is configured to stop the blood treatment therapy and to raise an alarm when the flow rate of the at least one substitution solution pump (36, 56) falls below a predetermined value.

8. The blood treatment device (2) according to claim 7, **characterized in that** the predetermined value is set between 25 ml/h and 75 ml/h, in particular to approximately 50 ml/h.

9. The blood treatment device (2) according to any of the preceding claims, **characterized in that** the control unit (62) is configured to increase the flow rate of the substitution solution pump (36, 56) when the ultrafiltrate pressure returns to a normal pressure range.

10. The blood treatment device (2) according to any of the preceding claims, **characterized in that** the control unit (62) is configured to reduce the flow rate of the at least one substitution solution pump (36, 56) repeatedly when the ultrafiltrate pressure sensor drops repeatedly so as to maintain the ultrafiltrate pressure at a predetermined stable ultrafiltrate pressure or set it again to the predetermined stable ultrafiltrate pressure, until the flow rate of the at least one substitution solution pump (36, 56) falls below a predetermined value.

11. The blood treatment device (2) according to any of the preceding claims, **characterized by** a plurality, in particular two, substitution solution pumps (36, 56), the control unit (62) being configured to stop the blood treatment therapy and to raise an alarm when the flow rate of at least one of the substitution solution pumps (36, 56) falls below a predetermined value.

## Revendications

1. Dispositif de traitement du sang (2), en particulier machine de dialyse, pour l'utilisation dans des thérapies de traitement du sang, en particulier des thérapies de substitution du rein , avec :
une circulation sanguine (4) extracorporelle, un dialyseur (6) et un circuit de dialysat (8), dans lequel la circulation sanguine (4) extracorporelle et le circuit de dialysat (8) sont séparés l'un de l'autre par le biais d'une membrane (10) prévue dans le dialyseur (6), par le biais de laquelle du sang peut être filtré ;
au moins une pompe de solution de substitution (36, 56) qui est conçue afin de fournir une solution de substitution à la circulation sanguine (4) extracorporelle avant et/ou après le dialyseur (6) ;
un capteur de pression d'ultrafiltrat (42) qui est conçu afin de mesurer une pression dans le circuit de dialysat (8) après le dialyseur (6) ; et
un dispositif de commande (62) qui est conçu
afin de réduire automatiquement un débit de la au moins une pompe de solution de substitution (36, 56) lorsqu'une pression d'ultrafiltrat mesurée par le capteur de pression d'ultrafiltrat (42) diminue pendant une thérapie de traitement du sang en cours,
**caractérisé en ce que**
le dispositif de commande (62) est conçu afin de réduire le débit de la au moins une pompe de solution de substitution (36, 56) de telle manière qu'une pression d'ultrafiltrat stable soit obtenue, pression qui respecte au moins un écart prédéterminé par rapport à une valeur limite d'alarme de basse pression.

2. Dispositif de traitement du sang (2) selon la revendication 1, **caractérisé en ce que** le dispositif de commande (62) est conçu afin de constater qu'il existe une obturation du dialyseur (6) ou une coagulation du sang, et ce dans le seul but ou exclusivement sur la base que la pression d'ultrafiltrat mesurée par le capteur de pression d'ultrafiltrat (62) diminue pendant la thérapie de traitement du sang en cours.

3. Dispositif de traitement du sang (2) selon la revendication 2, **caractérisé en ce que** lors de la constatation qu'il existe une obturation du dialyseur (6) ou une coagulation du sang, une pression transmembranaire et/ou une résistance au flux du dialyseur (6) ne sont pas intégrées.

4. Dispositif de traitement du sang (2) selon l'une quelconque des revendications précédentes, **caractérisé en ce que** le dispositif de commande (62) est conçu afin d'ajuster dans le cas où il existe une obturation du dialyseur (6) ou une coagulation du sang, la pression d'ultrafiltrat dans le seul but ou exclusivement par une modification du débit de la au moins une pompe de solution de substitution (36, 56).

5. Dispositif de traitement du sang (2) selon l'une quelconque des revendications précédentes, **caractérisé en ce que** la pression d'ultrafiltrat stable est au moins de 50 mmHg au-dessus de la valeur limite d'alarme de basse pression.

6. Dispositif de traitement du sang (2) selon l'une quelconque des revendications précédentes, **caractérisé en ce que** le dispositif de traitement du sang (2) présente une surface d'utilisateur (64) comprenant un affichage avec un écran tactile et le dispositif de commande (62) est conçu afin d'afficher un avertissement sur l'affichage lorsque la pression d'ultrafiltrat diminue pendant la thérapie et le débit de la pompe de solution de substitution (36, 56) est réduit.

7. Dispositif de traitement du sang (2) selon l'une quelconque des revendications précédentes, **caractérisé en ce que** le dispositif de commande (62) est conçu afin d'arrêter la thérapie de traitement du sang et d'émettre une alarme lorsqu'un débit d'au moins une pompe de solution de substitution (36, 56) tombe sous une valeur prédéterminée.

8. Dispositif de traitement du sang (2) selon la revendication 7, **caractérisé en ce que** la valeur prédéterminée est définie entre 25 ml/h et 75 ml/h, en particulier à environ 50 ml/h.

9. Dispositif de traitement du sang (2) selon l'une quelconque des revendications précédentes, **caractérisé en ce que** le dispositif de commande (62) est conçu lorsque la pression d'ultrafiltrat revient dans une plage de pression normale afin d'augmenter le débit de la pompe de solution de substitution (36, 56).

10. Dispositif de traitement du sang (2) selon l'une quelconque des revendications précédentes, **caractérisé en ce que** le dispositif de commande (62) est conçu afin de réduire à plusieurs reprises ou de manière répétée lors d'une diminution à plusieurs reprises ou de manière répétée de la pression d'ultrafiltrat le débit de la au moins une pompe de solution de substitution (36, 56) afin de maintenir ainsi la pression d'ultrafiltrat pour une pression d'ultrafiltrat stable prédéterminée et de l'ajuster de nouveau à la pression d'ultrafiltrat stable prédéterminée, et ce jusqu'à ce que le débit de la au moins une pompe de solution de substitution (36, 56) tombe sous une valeur prédéterminée.

11. Dispositif de traitement du sang (2) selon l'une quelconque des revendications précédentes, **caractérisé par** plusieurs, en particulier deux, pompes de solution de substitution (36, 56), dans lequel le dispositif de commande (62) est conçu afin d'arrêter la thérapie de traitement du sang et d'émettre une alarme lorsqu'un débit d'au moins une parmi les pompes de solution de substitution (36, 56) tombe sous une valeur prédéterminée.
